# EUROPEAN PATENT APPLICATION

(11) **EP 4 560 642 A1**
(43) Date of publication of application: **28.05.2025**
(21) Application number: 24212617.5
(22) Date of filing: 13.11.2024
(51) Int. Cl.: G16H 20/30, A61B 5/00, G16H 40/63, G16H 50/20, G16H 50/30

(54) **A METHOD FOR PLANNING LOAD VALUES FOR A TARGET HORMONAL RESPONSE**

(30) Priority: 17.11.2023 FI 20236278
(71) Applicant: Summa Finland Oy, 90620 Oulu (FI)
(72) Inventor: Kinnunen, Tommi, 00190 Helsinki (FI); Juurikka, Aleksi, 91300 Oulu (FI); Kurek, Wojciech, 50-202 Wroclaw (PL); Nissilä, Juuso, 90230 Oulu (FI); Mäkinen, Marko, 90500 Oulu (FI)
(74) Representative: Moosedog Oy

(57) **Abstract**

Disclosed is a method for planning load values for a targeted hormonal response. The method comprising measuring, by load sensor (502), load values; receiving, by processor (504), a sequence of load values (202) to determine corresponding hormonal response values (204); presenting, by using a display (506), on a user interface, the sequence of load values and the corresponding hormonal response values; altering a concerned load value, from in-between the sequence of load values, to determine, by processor, one or more altered load values and corresponding altered hormonal response values subsequent to the concerned load value; presenting, by using the display, on the user interface, unaltered load and hormonal response values prior to the concerned load value and the altered load and hormonal response values subsequent to the concerned load value, wherein the altered and unaltered hormonal response values collectively define the targeted hormonal response.

## Description

### TECHNICAL FIELD

The present disclosure relates to methods for planning load values for targeted hormonal responses. Moreover, the present disclosure relates to systems for planning load values for targeted hormonal responses.

### BACKGROUND

Typically, in performing a given activity (i.e., physical, or mental), a person experiences a certain level of load which can be a physical load or a mental load, based on the activity that is performed. Subsequent to performing the given activity, a certain hormone response is generated in the person's body corresponding to the certain level of load. There are present solutions that measure the certain level of load and the corresponding hormone response to analyse efficiency of performing the given activity by the person.

Although, the present solutions fail to provide a way to create a plan for performing the given activity in future and determine what will be the corresponding hormone responses that will be generated in the person's body, in order to achieve a predetermined target in performing the given activity. Moreover, the present solutions fail to provide a way to determine and analyse effects and impact of changing a created plan for performing the performing the given activity.

Therefore, in light of the foregoing discussion, there exists a need to overcome the aforementioned drawbacks.

### SUMMARY

The aim of the present disclosure is to provide a method and a system to accurately plan load values by effectively optimizing load values to achieve a targeted hormonal response. The aim of the present disclosure is achieved by a method and a system for planning load values for a targeted hormonal response as defined in the appended independent claims to which reference is made to. Advantageous features are set out in the appended dependent claims.

Throughout the description and claims of this specification, the words *"comprise", "include", "have",* and *"contain"* and variations of these words, for example *"comprising"* and *"comprises",* mean *"including but not limited to",* and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF THE DRAWINGS

FIG. 1 is an illustration of a flowchart depicting steps of a method for planning load values for a targeted hormonal response, in accordance with an embodiment of the present disclosure;
FIG. 2 is an illustration of a graphical representation of a normalized line graph for depicting sequence of load values and corresponding hormonal response values, in accordance with an embodiment of the present disclosure;
FIG. 3 is an illustration of a flowchart depicting steps for determining corresponding hormonal response values for a sequence of load values, in accordance with an embodiment of the present disclosure;
FIG. 4A-C are illustrations of graphical representations of normalized line graphs for depicting sequence of load values and corresponding hormonal response values, in accordance with an embodiment of the present disclosure; and
FIG. 5 is a schematic illustration of a system for planning load values for a targeted hormonal response, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, the present disclosure provides a method for planning load values for a targeted hormonal response, the method comprising:
measuring, by a load sensor, load values;
receiving, by a processor, a sequence of load values to determine corresponding hormonal response values;
presenting, by using a display, on a user interface, the sequence of load values and the corresponding hormonal response values;
altering a concerned load value, from in-between the sequence of load values, to determine, by the processor, one or more altered load values and corresponding altered hormonal response values subsequent to the concerned load value;
presenting, by using the display, on the user interface, unaltered load and hormonal response values prior to the concerned load value and the altered load and hormonal response values subsequent to the concerned load value, wherein the altered and unaltered hormonal response values collectively define the targeted hormonal response.

The present disclosure provides an aforementioned method. The method significantly improves an accuracy and efficiency of planning the load values for achieving the targeted hormonal response. Moreover, the method successfully provides a way to determine and analyze how altering the concerned load value impacts the load values and the corresponding hormonal response values subsequent to the concerned load value. Furthermore, the method successfully enables the user to determine how to alter the concerned load value to achieve the targeted hormonal response value.

In a second aspect, the present disclosure provides a system for planning load values for a targeted hormonal response, the system comprising:
a load sensor for measuring load values;
a processor communicably coupled to the load sensor and operable to determine corresponding hormonal response values based on the load values, measured by the load sensor, wherein the processor is configured to:
   receive a sequence of load values to determine corresponding hormonal response values,
   alter a concerned load value, from in-between the sequence of load values, to determine one or more altered load values and corresponding altered hormonal response values subsequent to the concerned load value, and
   define the targeted hormonal response using the altered and unaltered hormonal response values; and
a display communicably coupled to the processor, wherein the display is configured to:
   present a user interface depicting the sequence of load values and the corresponding hormonal response values, and
   present, on the user interface, unaltered load and hormonal response values prior to the concerned load value and the altered load and hormonal response values subsequent to the concerned load value.

The present disclosure provides an aforementioned system. The system significantly improves an accuracy and efficiency of planning the load values for achieving the targeted hormonal response. Moreover, the system successfully provides a way to determine and analyze how altering the concerned load value impacts the load values and the corresponding hormonal response values subsequent to the concerned load value. Furthermore, the system successfully enables the user to determine how to alter the concerned load value to achieve the targeted hormonal response value. This has been found out to provide exceptional results for providing future estimations.

Throughout the present disclosure, the term *"load values"* refers to those values which quantify an extent of load experienced by a user at different instances of time while performing a given activity (such as running, walking, swimming, cycling, and the like). Notably, the load values are represented via measuring at least one physical or mental property associated with body of the user, such as at least one of: a heart rate, a blood pressure, an oxygen level, a mental stress, and the like. Herein, in an embodiment, the method comprises measuring, by a load sensor (described in detail below), the load values. For example, an oxygen level may be measured using load sensor implemented as an oximeter. It will be appreciated that a hormonal response is triggered in the body of the user in response to the load experienced by the user while performing the given activity. Optionally, the hormonal response is measured as levels of a given hormone (such as cortisol, testosterone, and the like) in the body of the user. Alternatively, the hormonal response is measured as a ratio of a level of a first hormone to a level of a second hormone in the body of the user. Throughout the present disclosure, the term *"targeted hormonal response"* refers to final hormonal response that the user aims to achieve in a future instance of time. Subsequently, to achieve the targeted hormonal response, planning the load values is required.

The method comprises receiving a sequence of load values to determine corresponding hormonal response values. Herein, in an embodiment, the method comprises receiving, by a processor (described in detail below), a sequence of load values are received to determine corresponding hormonal response values. Throughout the present disclosure, the term *"sequence of load values"* refers to the load values that needs to be performed by the user in a given sequence at given instances of time in order to achieve the targeted hormonal response. Notably, a part of the sequence of load values have been already experienced by the user for performing the given activity prior to receiving the sequence of load values, whereas another part of the sequence of load values will be experienced by the user for performing the given activity at predetermined future instances of time. Throughout the present disclosure, the term *"corresponding hormonal response values"* refers to the hormonal response values that are correspondingly determined for the sequence of load values, wherein a given corresponding hormonal response value indicates the hormonal response that is generated in the body of the user in response to a given load value from amongst the sequence of load values experienced by the user. Notably, for each load value from amongst the sequence of load values, a corresponding hormonal response value is determined. It will be appreciated that the corresponding hormonal response values are indicative of an effectiveness of the sequence of load values.

Moreover, the method comprises presenting, on a user interface, the sequence of load values and the corresponding hormonal response values. Herein, in an embodiment, the method comprises presenting, by using a display (described in detail below), on a user interface, the sequence of load values and the corresponding hormonal response values. Throughout the present disclosure, the term *"user interface"* refers to a space where interaction between the user and the display occurs. Typically, the user interface presents the sequence of load values and the corresponding hormonal response values. Beneficially, presenting the sequence of load values and the corresponding hormonal response values on the user interface enables the user to view and interpret how the targeted hormonal response value will be achieved by the user via the sequence of load values and the corresponding hormonal response values. Moreover, the user interface is designed to allow the user to interact easily, efficiently, and in a user-friendly manner with the display for altering the concerned load value.

Furthermore, the method comprises altering a concerned load value, from in-between the sequence of load values, to determine one or more altered load values and corresponding altered hormonal response values subsequent to the concerned load value. Herein, in an embodiment, the method comprises altering a concerned load value, from in-between the sequence of load values, to determine, by the processor, one or more altered load values and corresponding altered hormonal response values subsequent to the concerned load value. In other words, the processor is configured to determine one or more altered load values and corresponding altered hormonal response values subsequent to the concerned load value that is altered. Throughout the present disclosure, the term *"concerned load value"* refers to that load value from amongst the sequence of load values that the user wants to alter according to user's preference. Notably, the concerned load value is altered by increasing or decreasing the concerned load value. It will be appreciated that the concerned load value is selected from the another part of the sequence of load values that will be experienced by the user for performing the given activity at the predetermined future of instances of time. Throughout the present disclosure, the term *"altered load value"* refers to that load value in the sequence of load values subsequent to the concerned load value, which is determined by alteration due to alteration in the concerned load value. It will be appreciated that the *"one or more altered load values"* refers to a *"single load value"* in some implementations and to a *"plurality of load values"* in other implementations. Notably, altering the concerned load value impacts all the load values that are subsequent to the concerned load value in the sequence of load values. Subsequently, all the load values that are subsequent to the concerned load value in the sequence of load values are altered to determine the one or more altered load values. Throughout the present disclosure, the term *"corresponding altered hormonal response values"* refers to those hormonal response values that are correspondingly determined after alteration in the one or more altered load values.

Furthermore, the method comprises presenting, on the user interface, unaltered load and hormonal response values prior to the concerned load value and the altered load and hormonal response values subsequent to the concerned load value, wherein the altered and unaltered hormonal response values collectively define the targeted hormonal response. Herein, in an embodiment, the method comprises presenting, by using the display, on the user interface, unaltered load and hormonal response values prior to the concerned load value and the altered load and hormonal response values subsequent to the concerned load value, wherein the altered and unaltered hormonal response values collectively define the targeted hormonal response. Throughout the present disclosure, the term *"unaltered load and hormonal response values"* refers to those load values and hormonal response values that are prior to the concerned load value which remains unaffected even after altering the concerned load value. Notably, presenting the unaltered load and hormonal response values prior to the concerned load value and the altered load and hormonal response values subsequent to the concerned load value, on the user interface enables the user to know how altering the concerned load value alters the load and hormonal response values subsequent to the concerned load value. Beneficially, the user is able to effectively determine what alteration to make to the concerned load value based on the unaltered load and hormonal response values prior to the concerned load value and the altered load and hormonal response values subsequent to the concerned load value, presented, on the user interface.

Moreover, the user interface comprises a normalized line graph for depicting the altered and unalerted load values and corresponding altered and unaltered hormonal response values, and wherein a pair of altered load value and corresponding altered hormonal response value or a pair of unaltered load value and corresponding unaltered hormonal response value is represented together using a point in the normalized line graph. Throughout the present disclosure, the term *"normalized line graph"* refers to a type of graphical data representation that represents two given variables over a continuous range of values. Optionally, the range of values in the normalized graph in the aforementioned method is between -100 to 100. Notably, the two given variables represented in the normalized line graph are the load values along a horizontal x-axis and the corresponding hormonal response values along a vertical y-axis. The technical effect of the normalized line graph is that it is designed to visually represent the relationship between load values and their corresponding hormonal response values, where the x-axis of the graph plots the load values, while the y-axis plots the corresponding hormonal response values, which allows the user to visualize the effects of changes in load values on hormonal responses in a clear, simultaneous manner.

Subsequently, the point in the normalized line graph comprises a pair of the given load value and the corresponding given hormonal response value, to represent both the given load value along the horizontal x-axis and the corresponding given hormonal response value along the vertical y-axis, simultaneously in the normalized line graph. Herein, in an embodiment, the point (or the single point) in the normalized line graph, presented on the display of the user interface, comprises a pair of the given load value and the corresponding given hormonal response value, to represent both the given load value along the horizontal x-axis and the corresponding given hormonal response value along the vertical y-axis, simultaneously in the normalized line graph. Optionally, the pair of the given load value and the corresponding given hormonal response value represented by the point may be the pair of altered load value and corresponding altered hormonal response value. Alternatively, the pair of the given load value and the corresponding given hormonal response value represented by the point may be the pair of unaltered load value and corresponding unaltered hormonal response value. A technical effect of the user interface comprising the normalized line graph is that the altered and unalerted load values and corresponding altered and unalerted hormonal response values are presented to the user in an organized and informative manner that provides improved insights to the user about the impact of altering the concerned load value on the altered load values and corresponding altered hormonal response values.

Moreover, the technical effect of plotting given load value along the horizontal x-axis and the corresponding given hormonal response value along the vertical y-axis, simultaneously in the normalized line graph, is that the method provides a clear and intuitive visual representation of the relationship between a given load value and its corresponding hormonal response on a normalized line graph. By plotting the load value on the horizontal x-axis and the hormonal response value on the vertical y-axis, the method allows users to simultaneously observe and analyze how specific load values impact hormonal responses. This real-time visualization aids in the optimization and adjustment of load sequences, facilitating more efficient planning and decision-making. The feature enables users to easily identify trends, deviations, or improvements in hormonal responses based on load modifications, leading to more precise control over desired physiological outcomes.

Optionally, the normalized line graph comprises a plurality of points defined based on the sequence of load values, and wherein a pair of subsequent points of the plurality of points is connected using a solid line or a dotted line. In this regard, each load value from amongst the sequence of load values is represented as a given point from amongst the plurality of points in the normalized line graph. Notably, each pair of subsequent points from amongst the plurality of points are joined to form lines in the normalized line graph. Herein, each line formed by joining the pair of subsequent points is either the solid line or the dotted line.

Optionally, the solid line represents a past load event, associated with the sequence of load values, corresponding to measured load values and corresponding hormonal response values depicted by a pair of subsequent points in the normalized line graph. Throughout the present disclosure, the term *"past load event"* refers to that load event that has occurred in a past instance of time corresponding to a pair of measured load values and corresponding hormonal response values. Subsequently, the pair of the measured load values and corresponding hormonal response values are depicted by the pair of the subsequent points of the solid line that depict the pair of the measured load values and corresponding hormonal response values in the normalized line graph. A technical effect is that the user is able to effectively analyze the past load events in the normalized line graph for improvement in planning the load values for the targeted hormonal response.

Optionally, the dotted line represents a future load event, associated with the sequence of load values, corresponding to load values and corresponding hormonal response values to be measured and depicted by a pair of subsequent points in the normalized line graph. Throughout the present disclosure, the term *"future load event"* refers to that load even that will take place in a future instance of time, for which a pair of load values and corresponding hormonal response values are to be measured. Subsequently, the pair of the load values and corresponding hormonal response values that are to be measured are depicted by the pair of the subsequent points of the solid line that depict the pair of the measured load values and corresponding hormonal response values in the normalized line graph. A technical effect is that the user is able to effectively analyze the future load events in the normalized line graph for improvement in planning the load values for the targeted hormonal response.

Optionally, the targeted hormonal response is an end point, of the normalized line graph, associated with one of the altered or unaltered hormonal response values corresponding to a last load value of the sequence of load values. In this regard, the targeted hormonal response is the final hormonal response value that the user wants to generate in their body in response to the last load value of the sequence of load values. Throughout the present disclosure, the term *"last load value"* refers to that load value that is experienced by the user while performing the given activity for a last time. Notably, the end point represents the one of the altered or unaltered hormonal response values along the y-axis, corresponding to the last load value of the sequence of load values along the x-axis. A technical effect is that the user is able to easily check whether the targeted hormonal response depicted in the normalized line graph is according to user's preference or not.

Optionally, the altered and unaltered hormonal response values is associated with at least one of: an anabolic hormone level, a catabolic hormone level, a ratio of anabolic to catabolic hormone levels. Throughout the present disclosure, the term *"anabolic hormone level"* refers to a level of that hormone in the user's body, which promotes synthesis and build-up of muscles, particularly proteins and other complex molecules in the user's body. Optionally, the anabolic hormone level may be measured as a level of testosterone in the user's body. Throughout the present disclosure, the term *"catabolic hormone level"* refers to a level of that hormone in the user's body, which promotes breakdown of complex molecules into simpler molecules in the user's body. Optionally, the catabolic hormone level may be measured as a level of cortisol in the user's body. Throughout the present disclosure, the term *"ratio of anabolic to catabolic hormone levels"* refers to a ratio of the level of the anabolic hormone to the level of the catabolic hormone in the user's body. Optionally, the ratio of the anabolic to catabolic hormone levels is measured as the ratio of the level of testosterone to the level of cortisol in the user's body. A technical effect is that the aforementioned method is suitable to use for different types of hormones.

Optionally, the altered and unaltered load value is associated with a Training Impulse value (TRIMP). Throughout the present disclosure, the term *"training impulse value (TRIMP)"* refers to a value of the load experienced by the user while performing a training session of a physical activity. Example unit of the TRIMP is amount of heart beats during the training. (Training volume, measured in minutes, and training intensity, measured as average heart rate (beats per minute or bpm)). As an example, for TRIMP value calculation: 50 min training at 140 bpm TRIMP = 50 × 140 = 7000. A technical effect of the altered and unaltered load value being associated with the TRIMP is that the aforementioned method is suitable to be applied for various different sports activities.

Optionally, determining the corresponding hormonal response values for the sequence of load values comprises:
determining a first hormonal response value using a first load value as an input, and
determining a second hormonal response value using a second load value and the first hormonal response value as an input, wherein the second load value is subsequent to the first load value of the sequence of load values.

In this regard, the term *"first load value"* refers to that load value that initiates the sequence of load values. Throughout the present disclosure, the term *"first hormonal response value"* refers to the hormonal response value that is determined in response to the first load value that is experienced by the user. Throughout the present disclosure, the term "second load value" refers to that load value which is measured after the first load value. Throughout the present disclosure, the term *"second hormonal response value"* refers to the hormonal response value that is determined in response to the second load value that is experienced by the user. Notably, for determining the second hormonal response value the first hormonal response value is also used as the input in addition to the second load value. A technical effect is that for determining each of the hormonal response value except the first hormonal response value corresponding to the given load value, a preceding hormonal response value corresponding to a preceding load value is also used as the input. Subsequently, each of the hormonal response value as the output of each altered load is plotted on the graph as discussed earlier. Furthermore, it is presented that this graph allows for the depiction of both altered and unaltered load and hormonal response values. The system dynamically updates the graph when any load value is altered, recalculating and plotting the corresponding hormonal response value. By displaying these pairs in real-time, the system provides a clear and intuitive view of the relationship between physical load adjustments and their hormonal impacts. This simultaneous representation of data points allows for efficient analysis, enabling users to track, compare, and optimize load adjustments for targeted hormonal responses in a precise and visually accessible manner. This way, the present disclosure focuses to easily identify trends, deviations, or improvements in hormonal responses based on load modifications, leading to more precise control over desired physiological outcomes by observing and analyzing specific load values and hormonal responses. The method disclosed in the present disclosure ensures planning load values to achieve a targeted hormonal response by first receiving a sequence of load values and determining their corresponding hormonal response values.

The present disclosure also relates to the system as described above. Various embodiments and variants disclosed above, with respect to the aforementioned method, apply mutatis mutandis to the system.

Throughout the present disclosure, the term *"load sensor"* refers to a sensing device that is capable of measuring data that is indicative of the load values experienced by the user while performing the given activity at different instances of time. Optionally, the load sensor comprises: a smartwatch, a fitness band, a smart ring. In this regard, the smartwatch, the fitness band and the smart ring comprises sensing elements which enable the smartwatch, the fitness band and the smart ring to measure the data that is indicative of the load values. A technical effect of the load sensor comprising: the smartwatch, the fitness band, the smart ring, is that a wide number of smart wearables or computing devices may be conveniently used as the load sensor. If the load value is related to mental load, then "sensor" can be considered to be a questionnaire. In that respect the load value (altered or unaltered) can be derived using questionaries (such as stress level, workload, personal issues, done / planned physical load) which the user provides answers. A load value related to the questionnaire is calculated using questions and answers. Beneficially, the load sensor not only provides physiological parameters but also psychological parameters of a subject, by being implemented as a questionnaire. The questionnaire may contain questions concerning such aspects that can not be measured by a physical sensing device.

Throughout the present disclosure, the term *"processor"* refers to a computational element that is operable to execute instructions of the system. Examples of the processor include, but are not limited to, a microprocessor, a microcontroller, a complex instruction set computing (CISC) microprocessor, a reduced instruction set (RISC) microprocessor, a very long instruction word (VLIW) microprocessor, or any other type of processing circuit. Furthermore, the processor may refer to one or more individual processors, processing devices and various elements associated with a processing device that may be shared by other processing devices. Additionally, one or more individual processors, processing devices and elements are arranged in various architectures for responding to and processing the instructions that execute the instructions of the system.

Throughput the present disclosure, the term *"display"* refers to a screen that virtually represents data in visual format. Optionally, the display may be selected from liquid crystal display (LCD) light-emitting diode (LED), backlit LCD, thin-film transistor (TFT) LCD, organic LED (OLED), Quantum dot (QLED) display, OLED display, AMOLED display, Super AMOLED display. Notably, the display is provided on the user interface of a device, such as a smartphone, a tablet, a laptop, and so on.

Optionally, the processor and the display are associated with a mobile computing device. In an embodiment, the processor and the display are integrated within a mobile computing device. Throughout the present disclosure, the term *"mobile computing device"* refers to an electronic device having computing capabilities, which is portable and can be easily carried by the user. A technical effect of the processor and the display being associated with or integrated within the mobile computing device is that the user is able to conveniently manage and control an operation of the aforementioned system via the mobile computing device which significantly enhances user experience.

Optionally, the processor is associated with a server arrangement and the display is associated with a mobile computing device. In an embodiment, the processor is functionally connected with a server arrangement and the display is integrated within a mobile computing device. Throughout the present disclosure, the term *"server arrangement"* refers to computational element that is operable to execute instructions of the system. A technical effect is that a processing load on the processor significantly reduces due to the association of the processor with the server arrangement.

Optionally, the user interface comprises a normalized line graph for depicting the altered and unalerted load values and corresponding altered and unalerted hormonal response values, and wherein a pair of altered load value and corresponding altered hormonal response value or a pair of unaltered load value and corresponding unaltered hormonal response value is represented together using a point in the normalized line graph.

Optionally, the processor is operable to determine the corresponding hormonal response values for the sequence of load values by:
determining a first hormonal response value using a first load value as an input, and
determining a second hormonal response value using a second load value and the first hormonal response value as an input, wherein the second load value is subsequent to the first load value of the sequence of load values.

Herein, the present disclosure discloses a method which involves planning load values to achieve a targeted hormonal response by first receiving a sequence of load values and determining their corresponding hormonal response values. These values are displayed on a user interface. A specific load value within the sequence can then be altered, and the system calculates the subsequent altered load values along with their corresponding hormonal response values. The user interface presents both the unaltered load and hormonal response values prior to the change and the altered values afterward. Collectively, these altered and unaltered values define the targeted hormonal response, providing a clear way to adjust and optimize the load values for desired hormonal outcomes.

In other words, the present disclosure provides a method for planning future loadings for next N events. In this first a starting a starting value load and a starting hormonal response is received. This can be received for example from a user interface or from a measurement device(es) (such as heart rate monitor and/or hormonal value measurement device). User interface is then provided on which a sequence of N load estimations is requested from the user. Algorithm is used to determine after this N hormonal responses related to the sequence of N load estimations. N can be any integer, but typically 7 for athletics. Those values are presented on an user interface i.e the sequence of load estimations and the determined set of hormonal responses are presented. User interface can be used by the user to simulate effect of different loadings in the future. This is done by providing an interface to modify a load estimation M from the sequence of N load estimations, wherein in M∈{1,...,N}. M is in practice any load between "last load estimation" and the first. As the load estimation M is modified then respective hormonal response is remodelled and all sequential after M up to N are also remodelled. Loadings of M+1 to N are kept same. User interface is than modified to present the modified sequence of N load estimations and related determined and re-determined hormonal responses. Technical effect of this is providing an easy way to plan future exercises (loads).

### DETAILED DESCRIPTION OF THE DRAWINGS

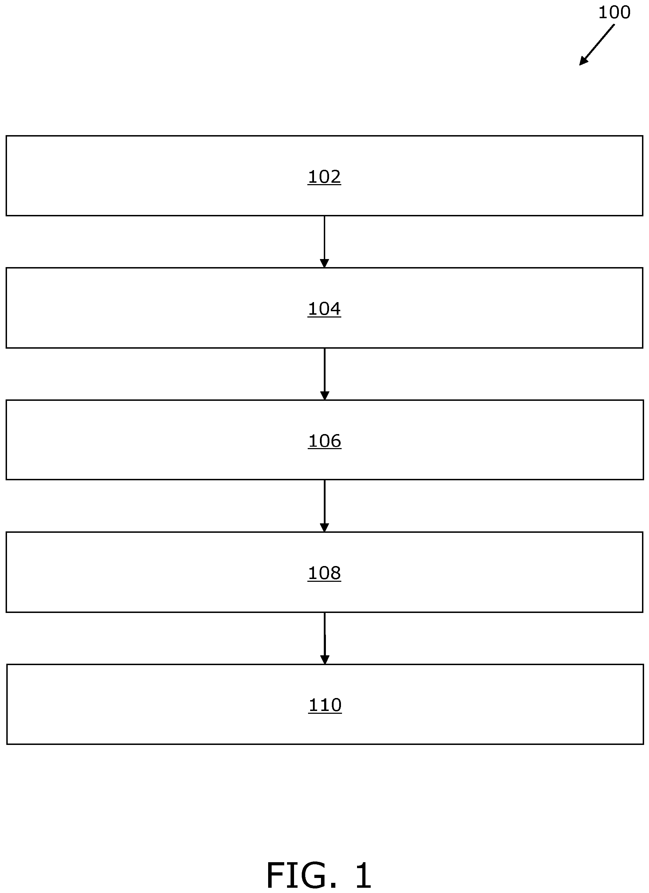

Referring to FIG. 1, illustrated is a flowchart **100** depicting steps of a method for planning load values for a targeted hormonal response, in accordance with an embodiment of the present disclosure. At step **102,** load values are measured by a load sensor. At step **104,** a sequence of load values are received to determine corresponding hormonal response values. Herein, the sequence of load values are received, by a processor, to determine corresponding hormonal response values. At step **106,** the sequence of load values and the corresponding hormonal response values are presented, on a user interface. Herein, the sequence of load values and the corresponding hormonal response values are presented, by using a display, on the user interface. At step **108,** a concerned load value is altered, from in-between the sequence of load values, to determine one or more altered load values and corresponding altered hormonal response values subsequent to the concerned load value. Herein, the concerned load value is altered, from in-between the sequence of load values, to determine, by the processor, one or more altered load values and corresponding altered hormonal response values subsequent to the concerned load value. At step **110,** unaltered load and hormonal response values prior to the concerned load value and the altered load and hormonal response values subsequent to the concerned load value are presented, on the user interface, wherein the altered and unaltered hormonal response values collectively define the targeted hormonal response. Herein, the unaltered load and hormonal response values prior to the concerned load value and the altered load and hormonal response values subsequent to the concerned load value are presented, by using the display, on the user interface, wherein the altered and unaltered hormonal response values collectively define the targeted hormonal response.

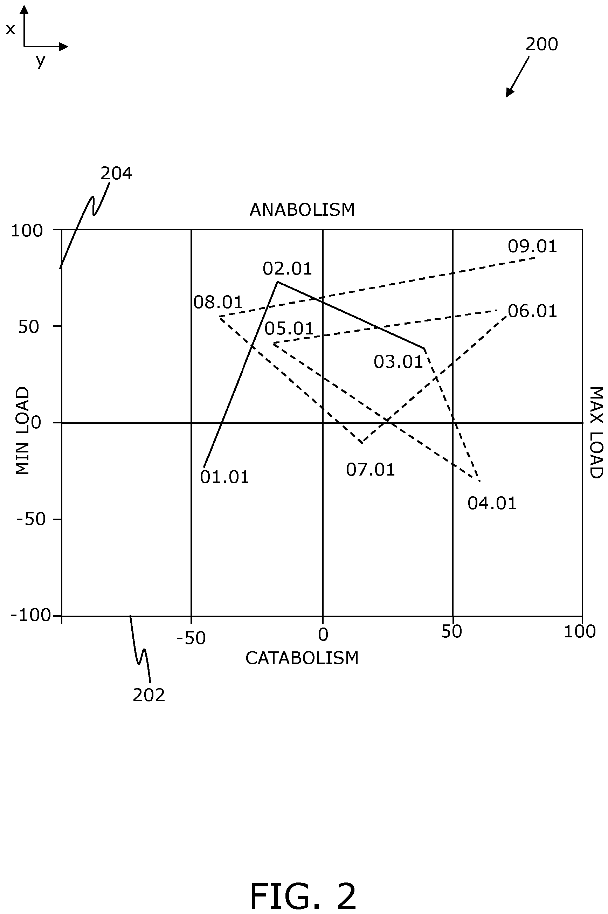

Referring to FIG. 2, illustrated is a graphical representation of a normalized line graph **200** for depicting sequence of load values **202** and corresponding hormonal response values **204,** in accordance with an embodiment of the present disclosure. As shown, an x-axis represents the sequence of load values **202** and a y-axis represents the corresponding hormonal response values **204.**

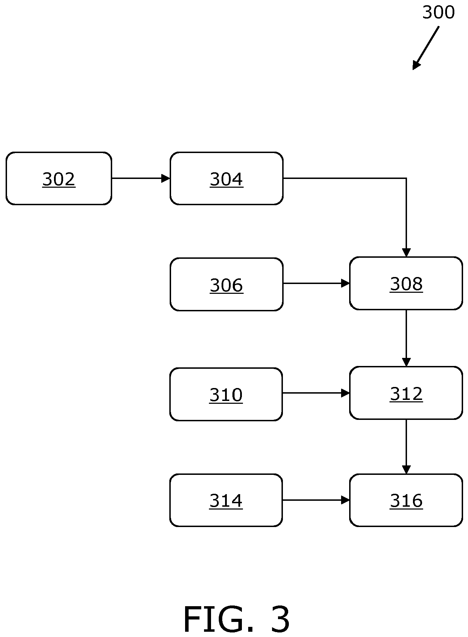

Referring to FIG. 3, illustrated is a flowchart **300** depicting steps for determining corresponding hormonal response values for a sequence of load values, in accordance with an embodiment of the present disclosure. At step **302,** a first load value of the sequence of load values is received. At step **304,** a first hormonal response value is determined using the first load value. At step **306,** a second load value of the sequence of load values is received. At step **308,** a second hormonal response value is determined using the second load value and the first hormonal response value as an input, wherein the second load value is subsequent to the first load value of the sequence of load values. At step **310,** a third load value of the sequence of load values is received. At step **312,** a third hormonal response value is determined using the third load value and the second hormonal response value as an input, wherein the third load value is subsequent to the second load value of the sequence of load values. At step **314,** a fourth load value of the sequence of load values is received. At step **316,** a fourth hormonal response value is determined using the fourth load value and the third hormonal response value as an input, wherein the fourth load value is subsequent to the third load value of the sequence of load values.

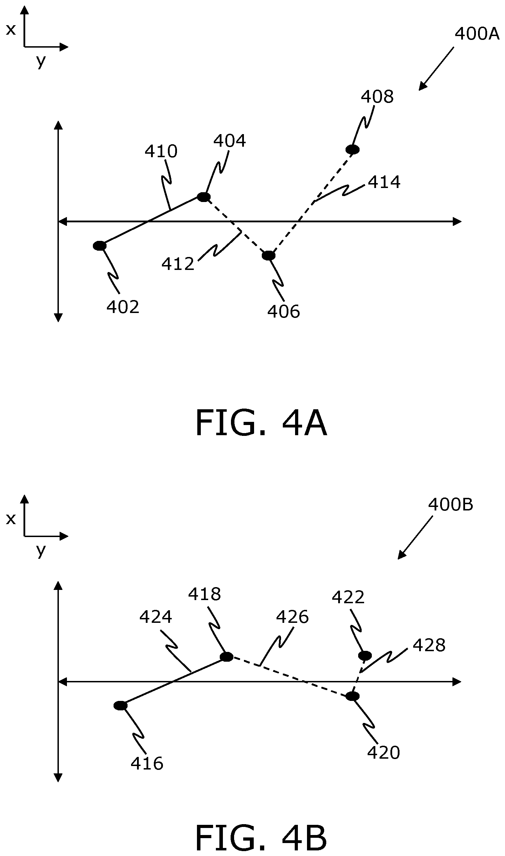

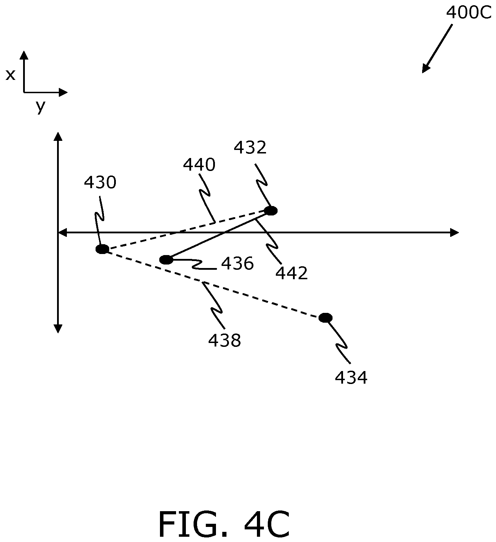

Referring to FIGs. 4A-C, illustrated are graphical representations of normalized line graphs **400A, 400B,** and **400C,** respectively, for depicting sequence of load values and corresponding hormonal response values, in accordance with an embodiment of the present disclosure. As shown, points **402, 404, 406,** and **408** represent pairs of a given load value and corresponding given hormonal response value in the normalized line graph **400A.** Moreover, a solid line **410** represents a past load event and dotted lines **412** and **414** represent future load events, respectively, in the normalized line graph **400A.** As shown, points **416, 418, 420,** and **422** represent pairs of a given load value and corresponding given hormonal response value in the normalized line graph **400B.** Moreover, a solid line **424** represents a past load event and dotted lines **426** and **428** represent future load events, respectively, in the normalized line graph **400B.** As shown, points **430, 432, 434,** and **436** represent pairs of a given load value and corresponding given hormonal response value in the normalized line graph **400C.** Moreover, a solid line **438** represents a past load event and dotted lines **440** and **442** represent future load events, respectively, in the normalized line graph **400C.** When looking cumulatively to FIG 4A, 4B and 4C we can see that first two points are same two last points are modelled. In given example load value of the last point is planned to be same in all scenarios. The user thus simulates what to do on day 3 (i.e. points **406, 420, 430).** "What to do" refers to a planned load. In Fig 4A user simulates situation with medium load. The simulation affects hormonal response associated with point **406** and hormonal response for load of the fourth day (point **408).** We can see that hormonal response is high on day four. In Fig 4B the user simulates higher load by sliding load to right in respect to axis. It can be seen that related hormonal response is closer to "zero" point and also that in day four hormonal response would be lower than in scenario of fig 4A. In fig 4C user tries unexpected scenario of minimizing load (for example resting entire day). Hormonal response value goes down for said data point **430** but also for day four as indicated **434.** This way user can plan day three loadings to have target hormonal response for the day four loading. As an example day four might be competition day and thus load will be maximum load and based on historical data we know which hormonal response value is best for said user. For example in given example user might know that as low value as possible is best thus the user elects to rest on day 3.

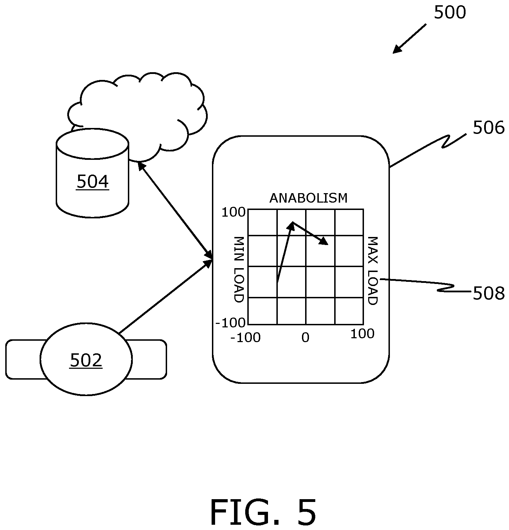

Referring to FIG. 5, illustrated is a schematic illustration of a system **500** for planning load values for a targeted hormonal response, in accordance with an embodiment of the present disclosure. As shown, the system comprising a load sensor **502** for measuring load values. Moreover, the system comprises a processor **504** communicably coupled to the load sensor **502** and operable to determine corresponding hormonal response values based on the load values, wherein the processor **504** is configured to receive a sequence of load values to determine corresponding hormonal response values; alter a concerned load value, from in-between the sequence of load values, to determine one or more altered load values and corresponding altered hormonal response values subsequent to the concerned load value, and define the targeted hormonal response using the altered and unaltered hormonal response values. Furthermore, the system comprises a display **506** communicably coupled to the processor **504,** wherein the display **506** is configured to present a user interface **508** depicting the sequence of load values and the corresponding hormonal response values, and present, on the user interface **508,** unaltered load and hormonal response values prior to the concerned load value and the altered load and hormonal response values subsequent to the concerned load value.

## Claims

1. A method for planning load values for a targeted hormonal response, the method comprising:
measuring, by a load sensor (502), load values;
receiving, by a processor (504), a sequence of load values (202) to determine corresponding hormonal response values (204);
presenting, by using a display (506), on a user interface, the sequence of load values and the corresponding hormonal response values;
altering a concerned load value, from in-between the sequence of load values, to determine, by the processor, one or more altered load values and corresponding altered hormonal response values subsequent to the concerned load value;
presenting, by using the display, on the user interface, unaltered load and hormonal response values prior to the concerned load value and the altered load and hormonal response values subsequent to the concerned load value, wherein the altered and unaltered hormonal response values collectively define the targeted hormonal response.

2. A method according to claim 1, wherein the user interface comprises a normalized line graph (200, 400A-C) for depicting the altered and unalerted load values and corresponding altered and unaltered hormonal response values, and wherein a pair of altered load value and corresponding altered hormonal response value or a pair of unaltered load value and corresponding unaltered hormonal response value is represented together using a point (402, 404, 406, 408, 416, 418, 420, 422, 430, 432, 434, 436) in the normalized line graph.

3. A method according to claim 2, wherein the normalized line graph (200, 400A-C) comprises a plurality of points defined based on the sequence of load values (202), and wherein a pair of subsequent points of the plurality of points is connected using a solid line (410, 424, 438) or a dotted line (412, 414, 426, 428, 440, 442).

4. A method according to claim 3, wherein the solid line (410, 424, 438) represents a past load event, associated with the sequence of load values (202), corresponding to measured load values and corresponding hormonal response values (204) depicted by a pair of subsequent points in the normalized line graph (200, 400A-C).

5. A method according to claim 3, wherein the dotted line (412, 414, 426, 428, 440, 442) represents a future load event, associated with the sequence of load values (202), corresponding to load values and corresponding hormonal response values (204) to be measured and depicted by a pair of subsequent points in the normalized line graph (200, 400A-C) .

6. A method according to claim 3, wherein the targeted hormonal response is an end point, of the normalized line graph (200, 400A-C), associated with one of the altered or unaltered hormonal response values corresponding to a last load value of the sequence of load values (202).

7. A method according to any of the preceding claims, wherein the altered and unaltered hormonal response values is associated with at least one of: an anabolic hormone level, a catabolic hormone level, a ratio of anabolic to catabolic hormone levels.

8. A method according to any of the preceding claims, wherein the altered and unaltered load value is associated with a Training Impulse value (TRIMP).

9. A method according to any of the preceding claims, wherein determining the corresponding hormonal response values (204) for the sequence of load values (202) comprises:
determining a first hormonal response value using a first load value as an input, and
determining a second hormonal response value using a second load value and the first hormonal response value as an input, wherein the second load value is subsequent to the first load value of the sequence of load values.

10. A system (500) for planning load values for a targeted hormonal response, the system comprising:
a load sensor (502) for measuring load values;
a processor (504) communicably coupled to the load sensor and operable to determine corresponding hormonal response values (204) based on the load values, measured by the load sensor, wherein the processor is configured to:
receive a sequence of load values (202) to determine the corresponding hormonal response values, and
alter a concerned load value, from in-between the sequence of load values, to determine one or more altered load values and corresponding altered hormonal response values subsequent to the concerned load value, and
define the targeted hormonal response using the altered and unaltered hormonal response values;
a display (506) communicably coupled to the processor, wherein the display is configured to:
present a user interface (508) depicting the sequence of load values and the corresponding hormonal response values, and
present, on the user interface, unaltered load and hormonal response values prior to the concerned load value and the altered load and hormonal response values subsequent to the concerned load value.

11. A system (500) according to claim 10, where the processor (504) and the display (506) are associated with a mobile computing device.

12. A system (500) according to claim 10, where the processor (504) is associated with a server arrangement and the display (506) is associated with a mobile computing device.

13. A system (500) according to any of the claims 10 to 12, wherein the load sensor (502) comprises at least one of: a smartwatch, a fitness band, a smart ring.

14. A system (500) according to any of the claims 10 to 13, wherein the user interface (508) comprises a normalized line graph (200, 400A-C) for depicting the altered and unaltered load values and corresponding altered and unalerted hormonal response values, and wherein a pair of altered load value and corresponding altered hormonal response value or a pair of unaltered load value and corresponding unaltered hormonal response value is represented together using a point (402, 404, 406, 408, 416, 418, 420, 422, 430, 432, 434, 436) in the normalized line graph.

15. A system (500) according to any of the claims 10 to 14, wherein the processor (504) is operable to determine the corresponding hormonal response values (204) for the sequence of load values (202) by:
determining a first hormonal response value using a first load value as an input, and
determining a second hormonal response value using a second load value and the first hormonal response value as an input, wherein the second load value is subsequent to the first load value of the sequence of load values.
